# EUROPEAN PATENT APPLICATION

(11) **EP 3 323 935 A1**
(43) Date of publication of application: **23.05.2018**
(21) Application number: 16199489.2
(22) Date of filing: 18.11.2016
(51) Int. Cl.: D06M 13/188, D06B 1/02, D06B 19/00, B01D 39/08, A61L 9/013, A61L 9/14, A62D 5/00, A61K 8/97, A61K 9/70, A61L 15/46

(54) **NATURAL ANTIMICROBIAL PINE ROSIN ADSORPTION APPARATUS**

(71) Applicant: Lignin Co., Ltd., Seoul 06163 (KR)
(72) Inventor: KIM, Lee Nam, Seoul, 06720 (KR)
(74) Representative: van Dam, Vincent

(57) **Abstract**

Disclosed herein is a technology for adsorbing a liquid pine rosin containing a pine rosin which is a natural antimicrobial substance, in a fiber woven raw cloth (10) or a nonwoven raw cloth (20) or a sewed and finished fiber product (300), wherein the disclosed pine rosin adsorption apparatus may include a storing container (80) for storing a liquid pine rosin (30), a liquid adjusting unit (100) for adjusting the input amount of the liquid pine rosin (30), a sealed spraying device (90) for spraying the liquid pine rosin in cooperation with the storing container (80), and a cold and warm air blowing device (120) for vaporizing an ethanol (110) contained in the liquid pine rosin (30), wherein the raw material of the liquid pine rosin (30) is adsorbed into the natural or synthetic fiber woven raw cloth (10) or the nonwoven raw cloth (20).

## Description

### Technical Field

The present invention relates to an adsorption technology, and in particular to a technology wherein a liquid pine rosin containing a pine rosin which is a natural antimicrobial substance can be adsorbed using a fiber woven raw cloth or a nonwoven raw cloth or a sewed fiber product.

### Background Art

When manufacturing a synthetic woven fabric or a natural pure cotton, a silk or pulp fiber woven fabric or a yarn of a nonwoven fiber raw cloth of a natural pump pure cotton, a fiber yarn, in general, is manufactured by adding a functional compound or a fiber product having an increased antibiosis and functionality has been developed in such a way to coat a synthetic functional substance on the thusly manufactured fiber product.

A mask, in general, is manufactured by adding a disinfectant after a synthetic fiber raw cloth having an antibiosis when manufacturing a conventional clothes or a mask fiber raw cloth has been manufactured, and then clothes, in particular, a mask product has been finished. In case of a disinfectant which is added to an artificial and natural fiber product, the added disinfectant may vaporize as time passes, so the effects thereof may disappear.

Meanwhile, as a conventional technology related with the present invention, there are the international patent application number 2006/011541 (laid-open on February 2, 2006) which describes an air filter and an apparatus and method for processing air using the same wherein a natural sap component extracted from a ginkgo tree is loaded, and the Korean patent laid-open number 10-2013-00222292 (laid-open on March 6, 2013) which describes a method for manufacturing an embossed wall paper using a nature friendly material.

Moreover, there is the Korean patent registration number 10-1254137 (the registration date is April 8, 2013) which is invented by the same inventor as the inventor of the present application and describes an oily pine rosin liquid of an environment friendly cosmetic material and a method for manufacturing a water pine rosin liquid.

As a characteristic of a natural pine rosin substance, a person having ordinary skill in the art is well aware that a natural pine rosin does not affect other substances, and even when a microorganism or a bacteria contacts with a pine rosin, the bacteria cannot survive. Even though harmful insects, the microorganism of a plant or various fungus bodies penetrate into a natural antibiosis substance, the pine rosin does not change, and under a contaminated environment, the pine rosin may turn into an amber jewel without being spoiled under soil for a long time, and it is known that the pine rosin emits Phytoncide which is a defending substance from a harmful insect a woody plant basically has, and the pine rosin has a natural chemical component to deodorize bad smells.

Moreover, when manufacturing a conventional synthetic woven fabric or a natural pure cotton, a silk or pulp fiber woven fabric or a yarn of a nonwoven fiber raw cloth of a natural pump pure cotton, a fiber yarn, in general, is manufactured by adding a functional compound or a fiber product having an increased antibiosis and functionality has been developed in such a way to coat a synthetic functional substance on the thusly manufactured fiber product. Clothes, an antimicrobial filter, an antimicrobial mask, etc. have been manufactured using these fiber raw cloths and nonwoven raw cloths. The thusly manufactured products are used for a medical purpose, an industrial purpose, a sanitary purpose, and a military purpose.

In addition, the conventional technology may cause an inconvenience since a user may feel repulsive with respect to a disinfection smell and may feel sick due to the presence of an artificial scent or disinfectant contained in the finished product. Moreover, if the artificial disinfectant component is exposed to the air, the components thereof might be quickly vaporized, so the antimicrobial effects may disappear.

Furthermore, when manufacturing a mask product, a water and air purification filter product, a face sheet for a dry skin of a cosmetic mask pack, a slipping prevention fiber sheet product for an industrial facility purpose using a fiber woven raw cloth of a natural or synthetic fiber or a nonwoven fiber raw cloth, a disinfectant may be contained in the product and then may be packaged. In this case, if the disinfectant is overly added, it may hurt a human body, and if it is less added, it may be impossible to prevent the bacteria, thus causing a problem.

In case of the disinfectant contained in an air purification filter, a vehicle air purifier, an indoor and outdoor environment, a vehicle air conditioner filter, other products or a mask product, when it is used, for example, a face puts on a mask after the package is opened, the disinfectant may vaporize in the air, for which the desired disinfection cannot be carried out, thus causing a problem.

The intervals of the mask fiber raw cloths are too dense, which makes it hard to breathe when putting on the mask, and the user may feel suffocating due to the moisture from the user's mouth, and a skin trouble may be caused at the face due to the moisture inside the mask.

In case of micro-sized yellow dusts which are a big issue in recent years, the density of the dusts is dense, so the dusts may enter into the respiratory system through the small spaces in the woven cloths, which is harmful to the health of the human body. A mask, an air purifier, an air conditioner, and a filter which are newly developed in the form of a filter so as to resolve such problems, are made of a nonwoven raw cloth material wherein the tissue density of the fiber woven cloth is dense.

Meanwhile, a pine rosin component is a natural raw material the safety of which has been proved according to the international cosmetic raw material handbook, so it is possible to manufacture a product which is able to improve the face skin problem. The pine rosin is a natural material, wherein the safety thereof with respect to the health of a human body has been proved, and the pine rosin may be applicable to various industry fields. In addition, if the pine rosin is liquefied and added to the raw cloth, the raw cloth may get impurities due to the sticky property of the pine rosin, and the raw cloths may stick to each other, and the user's hand or face may be sticky due to the pine rosin, for which the pine rosin cannot be directly used at a product.

### Disclosure of Invention

Accordingly, it is an object of the present invention to provide a pine rosin adsorption technology wherein the sticky level of a liquid pine rosin raw material is adjusted. In this state, even if a liquid pine rosin is adsorbed into a multi-ply raw cloth, the raw cloths cannot stick to each other and are not sticky, wherein there is not any moisture. The present invention is referred to a pine rosin adsorption technology wherein in case of a mask, the mask may be a little sticky due to the room temperature humidity when the user exhales warm air.

It is another object of the present invention to provide a liquid pine rosin adsorption technology wherein the pine rosin may become tangled or separable based on the contents of purified terpene oil contained in the pine rosin. If the liquid pine rosin manufactured after the sticky property of the liquid pine rosin raw material has been changed, is adsorbed into a fiber raw cloth or a filter raw cloth, there is not any sticky feeling and moisture. Even when the pine rosin is adsorbed into a multi-ply raw cloth, the raw cloths don't stick to each other, and they can be separated independently.

It is further another object of the present invention to provide a liquid pine rosin adsorption technology wherein in case of the liquid pine rosin manufactured using the pine rosin and ethanol, the raw cloth separation is available based on the ratio of the contents of the added terpene oil, and the terpene oil can be vaporized from the pine rosin, and the pine rosin can be dissolved using the ethanol which is added as a solvent in such a way to use the pine rosin formed of a solid pine rosin acid.

To achieve the above objects, there is provided a natural antimicrobial pine rosin adsorption apparatus and method, wherein the pine rosin adsorption technology includes a rotation roller device which is referred to a facility device configured to operate in cooperation with a pine rosin adsorption facility device which is able to adsorb a liquid pine rosin using a natural fiber raw cloth or a synthetic fiber raw cloth or a nonwoven raw cloth for a filter, wherein the rotation roller device is formed of a raw cloth hanging roller configuration for hanging a raw cloth and an opposite configuration for hanging the finished raw cloth. Moreover, the raw cloth transfer device is formed of a plurality of rollers, a storing container for storing the liquid pine rosin and a liquid pine rosin spraying device referred to a technology for adsorbing a liquid pine rosin using a raw cloth are configured to operate in cooperation with the liquid resin storing container, and the spraying device is formed of a plurality of air blowing devices which are configured in a sealed type into which only a raw cloth can be inputted and are configured in a structure which may allow to input again the liquid remaining after the spraying, and the ethanol contained in the liquid pine rosin can be vaporized in such a way to cooperatively engage a liquid input amount adjusting unit.

To achieve the above objects, there is provided a natural antimicrobial pine rosin adsorption apparatus and method, wherein the fiber raw cloth is hung over a raw cloth hanging roller, and the raw cloth is inputted in the pine rosin liquid storing container, and the pine rosin is adsorbed into the raw cloth, and the pine rosin adsorption raw cloth is compressed by a compression roller via the rotation roller, and the pine rosin liquid adsorbed into the raw cloth is squeezed out, and the squeezed pine rosin liquid is inputted again into the pine rosin storing container, and the pine rosin raw cloth which has passed through the compression roller is passed through the air blowing transfer device and the drying device, through which a pine rosin adsorption raw cloth is manufactured.

According to another liquid pine rosin adsorption technology of the present invention, a plurality of rotation rollers are provided, and a raw cloth is inserted between the rotation rollers, and the raw cloth is passed, and if the raw cloth is transferred by a transfer device to a sealed sprayer which is able to spray a liquid pine rosin, an operation sensor senses it, and while the raw cloth is passed, a liquid adjusting unit adjusts the spraying amount in the sealed sprayer of the spraying device, and the pine rosin is adsorbed into the raw cloth, and the ethanol components contained in the pine rosin are vaporized using wind generated by the air blowing device which operates in cooperation with the above operations, thus manufacturing the product.

According to another liquid pine rosin adsorption technology of the present invention, the technology for adsorbing the pine rosin into the sewed fiber product is referred to a configuration wherein a mask product, an air purification, water, an industry and an air conditioner made using a filter product, and a product made of a filter component are hung after a product hanger has been installed at a pine rosin adsorption facility apparatus, and a liquid pine rosin is sprayed to both sides of the fiber product with the aid of a sealed type double-side spraying device of the pine rosin adsorption facility apparatus by means of an automatic transfer roller, and if the product is transferred to a cooperating air blowing device, the ethanol contained in the liquid pine rosin and moisture can be vaporized by means of strong wind from the air blowing device, thus manufacturing the product.

Moreover, the fiber product pine rosin manufacturing technology is provided according to the present invention, wherein the raw cloths of the fiber products manufactured in a multi-ply structure don't stick to each other, and the raw cloths are separated independently, and the aforementioned technology can be applied to various fiber products, and the natural antimicrobial pine rosin can be adsorbed into the fiber products manufactured according to the present invention, which is able to prevent the growth of bacterium.

In addition, another manufacturing technology according to the present invention is referred to a technology for manufacturing a manual pine rosin adsorption fiber raw cloth and a pine rosin adsorption fiber product, which are manufactured in such a way that a liquid pine rosin is stored in a storing container, and a liquid pine rosin is manually sprayed to the fiber woven raw cloth or the nonwoven raw cloth or the sewed and finished fiber product using a manual sprayer.

### Advantageous Effects of the Invention

The present invention is able to provide a natural or synthetic fiber woven raw cloth or a nonwoven raw cloth or a sewed and finished fiber product in each of which a liquid pine rosin material has been adsorbed.

### Brief Description of Drawings

Figure 1 is a view illustrating an example of the technology wherein a pine rosin is adsorbed into a fiber woven raw cloth or a nonwoven raw cloth according to the present invention.
Figure 2 is a view illustrating an example of the technology wherein a pine rosin is adsorbed into a fiber product.

### Best Modes for carrying out the invention

In the present invention, living pine tree leaves are pressed so as to extract a natural scent of a pine tree leave wherein a liquid pine rosin product is added as a main raw material, and an ethanol is used as a pine rosin and a pine tree leave scent extraction solvent, and it is added to the pine tree leaves, and the mixture is precipitated for 1 to 10 days, thus extracting a pine tree leave scent component, and 5∼10% of the pine tree leave scent is added to and mixed with the liquid pine rosin raw material, thus manufacturing a liquid pine rosin containing the scent of a pine tree. Moreover, in order to adjust the sticky property of the raw cloth, a liquid pine rosin is manufactured, which allows a unique sticky property of the pine rosin to be adjusted by adding and mixing 1∼5% of the terpene oil. The thusly manufactured liquid pine rosin is adsorbed into the fiber raw cloth, through which the pine rosin having a natural antimicrobial property can be manufactured.

Moreover, according to the present invention, a liquid pine rosin is sprayed and adsorbed into a fiber raw cloth using a spraying device, and the ethanol contained in the liquid pine rosin provided to the raw cloth is vaporized using an air blowing device, after which only a pine rosin component can be coated on the raw cloth. In this way, a pine rosin adsorption raw cloth can be manufactured, wherein the raw cloth can become stiff like a cloth soaked with a paste without any moisture or sticky feeling, and this raw cloth may maintain a well dried and spread state, which allows the air to be sucked or discharged freely without filling the spaces between the fibers of the raw cloth.

According to the pine rosin adsorption fiber raw cloth manufactured according to the present invention, an air input and output can be well carried out, and if a user's face has moisture due to the user's breathing when using the mask after it has been manufactured, the spaces between the fiber tissues of the raw cloth containing the pine rosin components may become sticky, which blocks small-sized yellow dusts from entering the spaces between the fibers, and each harmful bacteria mixed in the yellow dusts may stick to the sticky pine rosin, and the Phytoncide components referred to the defending substance components contained in the pine rosin are able to prevent the growth of the harmful bacteria and sterilize such components, thus securing stability, and since the components which are able to improve the skin are contained, a face skin can be improved with the aid of moisture during the use of the mask.

Furthermore, the pine rosin is a substance produced from a pine tree and has a Phytoncide component which is referred to a defending substance from a harmful insect for the sake of a self-protection of a woody plant. The pine rosin contains a component which makes clear the mind and a natural component which is good to a human body, for example, a skin protection substance. The pine tree is the most stable woody plant and has been eaten for a medical purpose from the old time, and it is being developed in the form of a natural cosmetic raw material and a medical medicine.

Moreover, the pine rosin fiber raw cloth may be used to manufacture an air purifier, a water purifier, a vehicle air conditioner antimicrobial filter, etc. It may be used to manufacture a sanitary mask, a medical mask, an industrial mask, a yellow dust mask, and a face sheet for a mask pack. In another method, a mask may be manufactured in such a way that a part of the cut pine rosin adsorption raw cloth is inserted in the filter inside the mask when manufacturing the mask.

The raw cloth containing a pine rosin component may be used to manufacture, using an antimicrobial natural substance, the mask products, for example, a sanitary mask, a medical mask, an industrial mask, a yellow dust mask, and a military mask. In addition, it may be used to manufacture a filter products, for example, an air purification filter, an industrial filter, a water purifier filter, a vehicle air purifier, an air conditioner filter, and an infant and old person diaper product. It may be used to manufacture an infant gauze product, for example, a Band-Aid, a bandage, a massage medical sheet, etc. The pine rosin is a natural material and is not harmful to a human body and has an antimicrobial function and a deodorization function to deodorize a bad smell, and the scent of a natural pine tree leave makes clear the mind.

In the pine rosin fiber raw cloth, a pine rosin liquid is adsorbed into the raw cloth, so air and moisture don't vaporize, and even when it is washed with water, the pine rosin component may remain at the fiber raw material for a predetermined time period, by which the antimicrobial effect can be maintained longer, and in case of the mask made using a fiber raw cloth or a fiber product containing a pine rosin component, since it is made using a raw cloth wherein the density interval between the woven tissue is large, it is easy for a user to breathe while the mask is put on, and the warm moisture from the user's mouth may make the pine rosin sticky, so when inhaling the air, the small-sized dusts can be stuck to the sticky pine rosin, thus preventing the inputs of the small-sized dusts. In this way, the suction of the yellow dusts can be reduced, and the growth of the harmful bacteria which has been stuck to the sticky pipe rosin can be prevented, and the pine rosin has a sterilization component.

For example, if a face sheet for a mask pack of a cosmetic is made and used, an oily component good to a dry skin can be maintained, which is effective to a skin improvement, and it can be applied to a woman's sanitary pad, an infant and old person diaper product, a bandage, a plaster attached to a skin, a gauze bandage, etc. which are made in the form of a fiber pad, and a fiber air purifier, and a water purifier, an air conditioner filter. These component may contain a natural antibiosis, thus preventing the growth of bacterium. For this reason, the present invention can be applied to a health-related industry.

The preferred embodiments of the present invention will be described with reference to the accompanying drawings. The accompanying drawings are provided for the sake of further understanding of the embodiments of the present invention together with the descriptions which are provided so as to describe the principles of the present embodiments. The present invention is not limited to the present embodiments.

The above described embodiments will be described in detail together with the drawings. Throughout the specification, the known functions and detailed descriptions on a part of the configuration will be omitted for the sake of simplified descriptions.

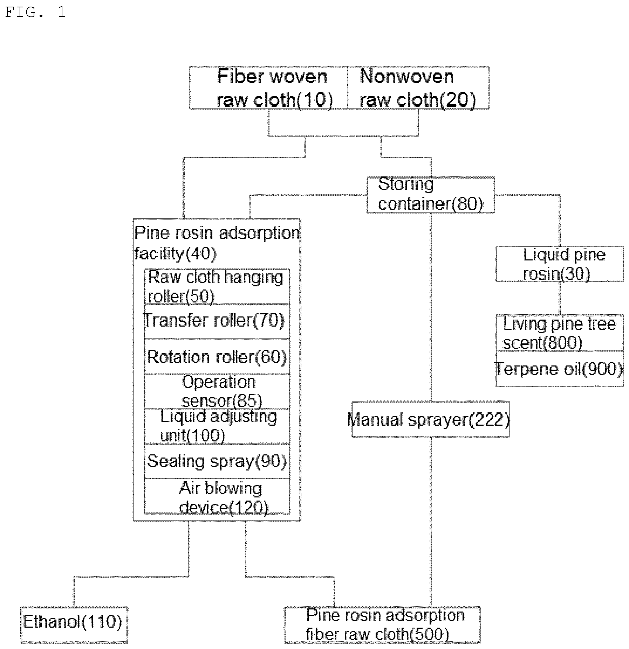

Figure 1 is a view illustrating an example of the pine rosin adsorption technology according to the present invention. Referring to Figure 1, the pine rosin adsorption apparatus may include, but is not limited to, a pine rosin adsorption facility 40 formed of a raw cloth hanging roller 50 configured to hang a natural or synthetic fiber woven raw cloth 10 or a nonwoven raw cloth 20, a rotation roller 60 around which a raw cloth is wound, a transfer roller 70 of a raw cloth transfer device configured to transfer a raw cloth, and an operation sensor 85 for detecting when the raw cloth passes through a sealed spraying environment. It may further include a storing container 80 for storing a liquid pine rosin 30, a liquid adjusting unit 100 for adjusting the input amount of the liquid pine rosin 30, a sealed spraying device 90 for spraying a liquid pine rosin in cooperation with the storing container 80, and a cold and warm air blowing device 120 for vaporizing the ethanol 110 contained in the liquid pine rosin 30. In this configuration, the raw material of the liquid pine rosin 30 can be adsorbed into the natural or synthetic fiber woven raw cloth 10 or the nonwoven raw cloth 20.

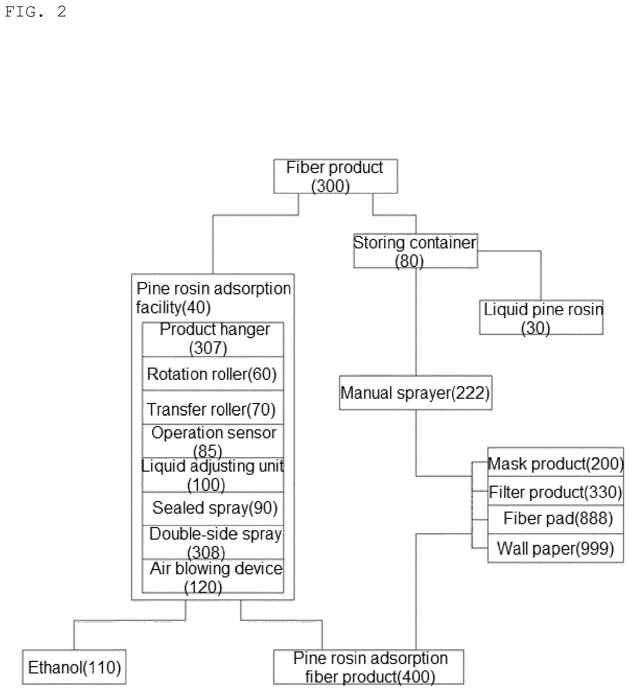

Figure 2 is a view illustrating another embodiment of the pine rosin adsorption technology according to the present invention. Referring to Figure 2, the pine rosin adsorption apparatus may include, but is not limited to, a pine rosin adsorption facility 40 which may be formed of a product hanger 307 for hanging a sewed and finished fiber product 300, a rotation roller 60 around which the sewed and finished fiber product 300 is wound, a transfer roller 70 of a transfer device for transferring a sewed and finished fiber product 300, and an operation sensor 85 for detecting when the sewed and finished fiber product 300 passes through the sealed spraying environment. Moreover, it may further include a storing container 80 for storing a liquid pine rosin 30, a liquid adjusting unit 100 for adjusting the input amount of the liquid pine rosin 30, a sealed spraying device 90 for spraying the liquid pine rosin in cooperation with the storing container 80, and a cold and warm air blowing device 120 for vaporizing the ethanol 110 contained in the liquid pine rosin 30. In this configuration, the raw material of the liquid pine rosin 30 can be adsorbed into the sewed and finished fiber product 300.

The method of the pine rosin adsorption technology according to another embodiment of the present invention may include, but is not limited to, hanging a natural or synthetic fiber woven raw cloth 10 or a nonwoven raw cloth 20 or a sewed and finished fiber product 300, rolling a raw cloth or sewed and finished fiber product 300, transferring a raw cloth or sewed and finished fiber product 300, and detecting when a raw cloth or sewed and finished fiber product 300 passes. Moreover, it may further include storing a liquid pine rosin in the storing container 80, adjusting the input amount of the liquid pine rosin 30, spraying the liquid pine rosin in cooperation with the storing container 80, and vaporizing the ethanol 110 contained in the liquid pipe rosin 30. The raw material of the liquid pipe rosin 30 may be adsorbed into the natural or synthetic fiber woven raw cloth 10 or the nonwoven raw cloth 20 or the sewed and finished fiber product 300.

More specifically, the device of the rotation roller 60 formed of a raw cloth hanging roller 50 for hanging the raw cloth and configured to roll the raw cloth may be provided as a facility apparatus which is configured to operation in cooperation with the configuration of the pine rosin adsorption facility with respect to the raw cloth in such a way to use as the raw material the pine rosin liquid wherein the living pine tree scent 800 and the terpene oil 900 are added, wherein the liquid pine rosin 30 has been dissolved in the ethanol 110 using the natural or synthetic woven raw cloth 10 or the synthetic fiber nonwoven raw cloth 20.

Moreover, a plurality of transfer rollers 70 are provided at the raw cloth transfer device. A sealed spraying device 90 is provided, which is able to spray a pine rosin liquid in cooperation with the storing container 80 configured to store the liquid pine rosin 30. The operation sensor 85 is provided, which is able to detect when the raw cloth passes through the sealed spraying environment. The sealed spraying device 90 which is able to spray a pine rosin liquid in cooperation with the storing container 80, may be formed of only a device wherein only the raw cloth can be inserted. The present invention may be configured in a structure wherein the liquid remaining after the spraying of the pine rosin liquid to the fiber raw cloth can be inputted again into the storing container 80. A plurality of the cold and warm air blowing devices 120 are provided, which are able to vaporize the ethanol 110 contained in the liquid pine rosin 30 in cooperation with the liquid adjusting unit 1100 with respect to the input amount of the liquid pine rosin 30, the configuration of which is referred to the technology of the manufacturing method of a natural antimicrobial pine rosin adsorption fiber raw cloth 500. The fiber products made using the pine rosin adsorption fiber raw cloth may be used to manufacture a mask product 200, a filter product 330, a fiber pad product 888, and a face sheet 305 for a cosmetic mask pack product.

Moreover, as the sewed and finished common fiber product 300, there may be a mask product 200, a filter product 330, a fiber pad product 888, and a face sheet product 305 for a cosmetic mask pack. These fiber products may be used for the sake of the product hanger 307 of the pine rosin adsorption facility 40. The fiber product to be adsorbed is engaged, and the sealed double-side spraying device 308 is provided, which is able to spray the pine rosin liquid when the product passes, and the operation sensor 85 which is able to indicate the operation, may be configured to cooperate, and the liquid adjusting unit 100 is provided to adjust the amount of the liquid pine rosin, and if the fiber product 300 passes through the sealed double-side spraying device 308, the liquid pine rosin 30 is automatically sprayed over both sides of the product, and the product is automatically transferred to the cold and warm air blowing device 120 which is configured to cooperate, and the ethanol 110 contained in the liquid pine rosin 30 and moisture are vaporized using a strong wind, thus finishing the natural antimicrobial pine rosin adsorption fiber product 400.

The present invention is able to provide the fiber raw cloth 500 containing a pine rosin and the pine rosin adsorption fiber product 400 which are manufactured by spraying a liquid pine rosin 30 to the fiber product 300 in such a way to use the sprayer which cooperates with the pine rosin storing container 80 using the manual sprayer with respect to the fiber woven raw cloth 10 and the nonwoven raw cloth 20 made using the liquid pine rosin 30.

In the present invention, the fiber raw cloth is rolled around the raw cloth hanging roller, and if the raw cloth is unrolled from the rotation roller and passes, the sprayer detection sensor detects, and the spraying device sprays an appropriate amount of the liquid pine rosin to the raw cloth, and if the raw cloth is transferred to the cooperating air blowing device, the strong wind generated by the air blowing device is projected toward the raw cloth, so the ethanol contained in the liquid pine rosin adsorbed into the raw cloth and moisture are vaporized, so the raw cloth can be dried and finished within any moisture, and the fiber raw cloth adsorbed into the pine rosin liquid is transferred to the cooperating raw cloth winding rotation roller, and the raw cloth is rolled around the raw cloth, thus finishing the product.

Moreover, the present invention is able to provide a fiber raw cloth which is able to protect a user's skin based on a harmful bacteria growth prevention function and a sterilization function and a manufacturing technology thereof, wherein the multi-ply raw cloths don't become tangled each other when adsorbing the pine rosin in another sewed and finished fiber product and can be separated independently, and the fiber product adsorbed into the pine rosin liquid can be used to manufacture a mask product, a filter product, a fiber pad product, a face sheet product for a cosmetic mask pack, etc., and the natural antimicrobial pine rosin can be adsorbed into the fiber raw cloth or the fiber product.

The applicant of the present invention herein attaches a test report which shows a result of the test after the product of the present invention has been submitted to the Korea Standard Test Research Center for the sake of tests. As seen therein, it shows the percentage of bacteriostatic (%) obtained using 7.2 x 10³ CFU/ml and 3.0 x 10⁶ CFU/ml of the concentrations of two inoculation bacteria liquids with respect to the sample 1: an air conditioner filter (an antimicrobial treatment); the sample 2: an air conditioner filter; the sample 3: an air purifier (an antimicrobial treatment), the sample 4: an air purifier; the sample 5: a water purifier; the sample 6: a water purifier (an antimicrobial treatment); the sample 7: a mask (an antimicrobial treatment); the sample 8: a mask; and the sample 13: a pipe tree. In this test report, the following percentages of bacteriostatic were obtained: the sample 1: an air conditioner filter (an antimicrobial treatment) has 99.9% and 98.9% of percentages of bacteriostatic; the sample 3: an air purifier (an antimicrobial treatment) has 99.9% and 99.4% of percentages of bacteriostatic; the sample 6: a water purifier (an antimicrobial treatment) has 99.9% and 99.9% of percentages of bacteriostatic; and the sample 13: a pine tree has 99.9% and 99.9% of percentages of bacteriostatic.

### [Test Report]

### KSTR, KOREAN STANDARD TEST RESEARCHER

Company: Lignin Purpose of use: quality control
Address: 516, Bongeunsa-ro, Gangnam-gu,
   Seoul, Korea
Person: Lee-nam, Kim
Name of sample: liquid resin filter, liquid resin

**Test result**

| **Test item** | | **Sample 1** | **Sample 2** | **Sample 3** | **Sample 4** | **Sample 5** | **Sample 6** | **Sample 7** | **Sample 8** | **Sample 13** |
|---|---|---|---|---|---|---|---|---|---|---|
| KS K 0693:2011 | | | | | | | | | | |
| *Staphylococcus aureus* ATCC 6538 | Percent of bacterio-static (%) | 99, 9 | 44,4 | 99, 9 | 30,6 | 55, 6 | 99, 9 | 99, 9 | 0 | 99, 9 |
| Concentration of inoculum | 7,2 x 10⁵ CFU/mL | | | | | | | | | |
| *Klebsiella pneumonia* ATCC 4352 | Percent of bacterio-static (%) | 98, 9 | 0 | 99,4 | 0 | 10,2 | 99, 9 | 9, 1 | 5,1 | 99, 9 |
| Concentration of inoculum | 3,0 x 10⁶ CFU/mL | | | | | | | | | |

| | | | | | | | | | | |
|---|---|---|---|---|---|---|---|---|---|---|
| • Control fabric: ISO 105 F02 Cotton | | | | | | | | | | |

### Industrial Applicability

In the pine rosin-contained raw cloth and pine rosin-adsorbed fiber product, the raw material is a pine rosin extracted from a pine tree, which is a natural antimicrobial substance. A small-sized yellow dust intake reduction effect can be obtained using a sticky function of a pine rosin along with a skin protection based on a moisture and a bad smell deodorization effect if a mask is manufactured using such a material. A harmful bacteria growth inhibition effect can be obtained. For a filter product, it may be applied to an air purification, an indoor and vehicle air conditioner, a water purifier filter product, etc. Since the present invention can be applied for a variety of purposes, the export industry may be promoted, and the natural antimicrobial pipe rosin-adsorbed fiber raw cloth and fiber product are effective to a harmful bacteria growth proliferation and a skin protection. If a natural scent component is added to a liquid pine rosin, the ethanol contained in the liquid pine rosin will be vaporized, after which the natural scent component may remain in the pine rosin and may be solidified in the fiber raw cloth. In this way, the vaporization of the scent can be prevented, and the fiber product is able to emit natural scents for a long time.

### Legend of reference numbers

| | | | |
|---|---|---|---|
| 10: | Fiber woven raw cloth | 20: | Nonwoven raw cloth |
| 30: | Liquid pine rosin | 40: | Pine rosin adsorption facility |
| 50: | Raw cloth hanging roller | 60: | Rotation roller |
| 70: | Transfer roller | 80: | Storing container |
| 100: | Liquid adjusting unit | 110: | Ethanol |
| 120: | Air blowing device | 200: | Mask product |
| 300: | Fiber product | 330: | Filter product |
| 800: | Living pine tree scent | 900: | Terpene oil |
| 999: | Wall paper | | |

## Claims

1. A pine rosin adsorption apparatus, comprising a pine rosin adsorption facility (40) includes a raw cloth hanging roller (50) configured to hang a natural or synthetic fiber woven raw cloth (10) or a nonwoven raw cloth (20); a rotation roller (60) for rolling the raw cloth; a transfer roller (70) of a raw cloth transfer device configured to transfer the raw cloth; and an operation sensor (85) for detecting when the raw cloth passes through a sealed spraying environment;
wherein the pine rosin adsorption apparatus further comprises:
a storing container (80) for storing a liquid pine rosin (30);
a liquid adjusting unit (100) for adjusting the input amount of the liquid pine rosin (30);
a sealed spraying device (90) for spraying the liquid pine rosin (30) in cooperation with the storing container (80); and
a cold and warm air blowing device (120) for vaporizing an ethanol (110) contained in the liquid pine rosin (30), and
wherein the raw material of the liquid pine rosin (30) is adsorbed into the natural or synthetic fiber woven raw cloth (10) or the nonwoven raw cloth (20).

2. The apparatus of claim 1, wherein the liquid pine rosin (30) can be adsorbed into such a way to insert the whole portions of the fiber woven raw cloth (10) or the nonwoven raw cloth (20) into the storing container (80).

3. The apparatus of claim 1, wherein the fiber raw cloth (500) in which the liquid pine rosin is adsorbed, includes any product of a mask product for a yellow dust prevention, a medical purpose, a sanitary purpose, and a military purpose, a filter product for an air purifier, a water purifier, a home air conditioner, and a vehicle, a face sheet product for a cosmetic mask pack, and a fiber pad product of a woman's sanitary pad, an infant and old person diaper, a bandage, a gauze bandage, and a plaster attached to a skin.

4. The apparatus of claim 1, further comprising a double-side spraying device (308) which is configured to spray the liquid pine rosin (30) to both sides.

5. The apparatus of claim 1, wherein a living pine tree scent (800) or a terpene oil (900) is added to the liquid pine rosin (30), thus adjusting the living pine tree scent and the sticky property of the raw cloth.

6. A pine rosin adsorption apparatus, comprising a pine rosin adsorption facility (40) which includes a product hanger (307) for hanging a sewed and finished fiber product (300), a rotation roller (60) for rolling the sewed and finished fiber product (300), a transfer device (70) of a transfer device for transferring the sewed and finished fiber product (300), and an operation sensor (85) for detecting when the sewed and finished fiber product (300) passes through a sealed spraying environment;
wherein the pine rosin adsorption apparatus further comprises:
a storing container (80) for storing a liquid pine rosin (30);
a liquid adjusting unit (100) for adjusting the input amount of the liquid pine rosin (30);
a sealed spraying device (90) for spraying the liquid pine rosin (30) in cooperation with the storing container (80) ; and
a cold and warm air blowing device (120) for vaporizing an ethanol (110) contained in the liquid pine rosin (30), wherein the raw material of the liquid pine rosin (30) is adsorbed into the sewed and finished fiber product (300).

7. The apparatus of claim 6, wherein the liquid pine rosin (30) can be adsorbed into such a way to insert the whole portions of the sewed and finished fiber product (300) into the storing container (80).

8. The apparatus of claim 6, wherein the sewed and finished fiber product (300) includes a mask product (200), a filter product (330), a face sheet product (305) for a cosmetic mask pack, a fiber pad product (888), and various wall paper products (999).

9. The apparatus of claim 6, further comprising a double-side spraying device (308) which is configured to spray the liquid pine rosin (30) to both sides.

10. The apparatus of claim 6, wherein a living pine tree scent (800) or a terpene oil (900) is added to the liquid pine rosin (30), thus adjusting the living pine tree scent and the sticky property of the raw cloth.

11. A pine rosin adsorption method, comprising hanging a natural or synthetic fiber woven raw cloth (10) or a nonwoven raw cloth (20) or a sewed and finished fiber product (300);
rolling the raw cloth or sewed and finished fiber product (300);
transferring the raw cloth or sewed and finished fiber product (300); and
detecting when the raw cloth or sewed and finished fiber product (300) passes through a sealed spraying environment,
wherein the pine rosin adsorption method further comprises:
storing a liquid pine rosin in a storing container (80) ;
adjusting the input amount of a liquid pine rosin (30) ;
spraying the liquid pine rosin (30) in cooperation with the storing container (80); and
vaporizing an ethanol (110) contained in the liquid pine rosin (30), and wherein the raw material of the liquid pine rosin (30) is adsorbed into the natural or synthetic fiber woven raw cloth (10) or the nonwoven raw cloth (20) or the sewed and finished fiber product (300).

12. The method of claim 11, wherein the fiber raw cloth (500) in which the liquid pine rosin (30) is adsorbed, includes any product of a mask product for a yellow dust prevention, a medical purpose, a sanitary purpose, and a military purpose, a filter product for an air purifier, a water purifier, a home air conditioner, and a vehicle, a face sheet product for a cosmetic mask pack, and a fiber pad product of a woman's sanitary pad, an infant and old person diaper, a bandage, a gauze bandage, and a plaster attached to a skin.

13. The method of claim 11, further comprising spraying the liquid pine rosin (30) to both sides using a double-side spraying device (308).

14. The method of claim 11, wherein a living pine tree scent (800) or a terpene oil (900) is added to the liquid pine rosin (30), thus adjusting the living pine tree scent and the sticky property of the raw cloth.

15. The method of claim 11, wherein the sewed and finished fiber product (300) includes a mask product (200), a filter product (330), a face sheet product (305) for a cosmetic mask pack, a fiber pad product (888), and various wall paper products (999).
